# FASCICULE DE BREVET EUROPEEN

(11) **EP 2 089 536 B1**
(45) Date de publication et mention de la délivrance du brevet: **21.11.2012**
(21) Numéro de dépôt: 07870350.1
(22) Date de dépôt: 28.11.2007
(51) Int. Cl.: C12Q 1/56

(54) **METHODE DE MESURE FONCTIONNELLE DE L'ACTIVITE DE LA THROMBOMODULINE PLASMATIQUE**
VERFAHREN ZUR FUNKTIONSMESSUNG PLASMATISCHER THROMBODULIN-AKTIVITÄT
METHOD FOR THE FUNCTIONAL MEASUREMENT OF PLASMATIC THROMBODULINE ACTIVITY

(30) Priorité: 29.11.2006 FR 0610444
(43) Date de publication de la demande: 19.08.2009
(73) Titulaire: Diagnostica Stago, 92600 Asnières (FR)
(72) Inventeur: VAN DREDEN, Patrick, F-29690 Brennilis (FR); ROUSSEAU, Aurélie, F-93240 Stains (FR)
(74) Mandataire: Desaix, Anne
(86) Numéro de dépôt international: PCT/FR2007/001955
(87) Numéro de publication internationale: WO 2008/081094

(56) Documents cités:
- FR-A1- 2 689 640
- OHLIN A-K ET AL: "Soluble thrombomodulin activity and soluble thrombomodulin antigen in plasma." JOURNAL OF THROMBOSIS AND HAEMOSTASIS : JTH MAY 2005, vol. 3, no. 5, mai 2005 (2005-05), pages 976-982, XP002433567 ISSN: 1538-7933
- ASO Y ET AL: "Relationship between soluble thrombomodulin in plasma and coagulation or fibrinolysis in type 2 diabetes." CLINICA CHIMICA ACTA; INTERNATIONAL JOURNAL OF CLINICAL CHEMISTRY NOV 2000, vol. 301, no. 1-2, novembre 2000 (2000-11), pages 135-145, XP002433568 ISSN: 0009-8981
- ESPINOSA GERARD ET AL: "Vascular involvement in Behçet's disease: relation with thrombophilic factors, coagulation activation, and thrombomodulin." THE AMERICAN JOURNAL OF MEDICINE JAN 2002, vol. 112, no. 1, janvier 2002 (2002-01), pages 37-43, XP002433569 ISSN: 0002-9343
- MOHRI MITSUNOBU: "Fundamental and Clinical Cardiology" 2003, MARCEL DEKKER INC , (NEW THERAPEUTIC AGENTS IN THROMBOSIS AND THROMBOLYSIS) 46 , XP009083743 pp 323-340. Soluble Thrombomodulin

## Description

L'invention concerne le domaine de l'hémostase et en particulier les désordres de la coagulation.

La présente demande a pour objet une méthode de mesure de l'activité fonctionnelle de la thrombomoduline plasmatique dans un échantillon biologique.

La méthode selon l'invention est réalisée *in vitro* à partir d'un échantillon biologique, en particulier d'un échantillon de sang prélevé chez un patient.

La méthode selon l'invention permet la mesure de l'activité fonctionnelle de la thrombomoduline en milieu plasmatique.

La présente demande vise en particulier une méthode de détermination de l'activité fonctionnelle de la thrombomoduline en milieu plasmatique, ladite détermination étant par exemple réalisée au moyen d'une méthode chromogénique ou fluorométrique.

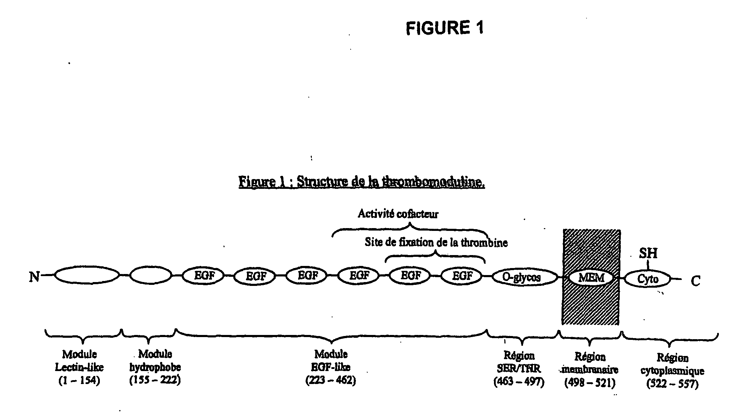

La thrombomoduline (TM) est un protéoglycane récepteur de la thrombine faisant partie intégrante de la membrane des cellules endothéliales. Responsable en partie de la propriété anticoagulante de l'endothélium, c'est une protéine de 557 acides aminés regroupés en trois parties : une partie C-terminale intra cytoplasmique impliquée dans les mécanismes d'*endocytose* ; une partie intra membranaire hydrophobe ; et une partie N-terminale constituée d'une longue chaîne peptidique. Cette dernière comprend quatre régions : une région riche en Ser/Thr, suivie d'une région de six domaines homologues à l'Epidermal Growth Factor (EGF-like), une région hydrophobe et enfin un domaine lectin-like. (Figure 1).

En 1981, Owen W.G. et Esmon C.T. caractérisent l'activité fonctionnelle de la thrombomoduline (Proceedings of the National Academy of Science USA, 1981, vol. 78, pages 2249-52 et Journal of Biological Chemistry 1981 vol. 256, pages 6632-5) mettant en évidence une activation de la protéine C plasmatique en protéine C activée anticoagulante, par la thrombine, catalysée par la thrombomoduline.

La séquence codante de la thrombomoduline humaine a été caractérisée et identifiée dans Wen D.Z. et al en 1987 (Biochemistry, 1987, vol. 26, pages 4350-7) décrite dans EMBL sous la référence BC 035602 et Weiler H. et al (J. Thromb. Haemost. 2003 Jul, 1(7) : 151-24).

La TM est présente à la surface de tous les vaisseaux sanguins (artères, veines, capillaires) et lymphatiques de l'organisme, avec une abondance particulière dans les organes richement vascularisés comme le placenta et le poumon. Elle est également exprimée par les kératinocytes, les ostéoblastes, les cellules mésangiales, les plaquettes et les neutrophiles.

La régulation de la synthèse de la TM est principalement sous la dépendance de l'AMP cyclique, du TNFα, de l'IL-1, et de l'acide rétinoïque.

La TM n'est pas secrétée par les cellules endothéliales, et n'est pas dégradée par la thrombine. Ce n'est que sous l'effet de différents types d'agressions de la cellule endothéliale (élastase des polynucléaires ; cytokines, macrophages, lipopolysaccharides...) que la TM endothéliale est clivée en fragments qui sont libérés dans la circulation sous forme soluble puis éliminés par voie rénale. La TM cellulaire une fois clivée est une forme soluble i.e., plasmatique, de TM, délétée des régions cytoplasmiques et membranaires (Ishiih et al, 1985, J. clin. Invest. 76, 2178). Le dosage de la TM plasmatique soluble (TMp) constitue un bon marqueur de lésion endothéliale.

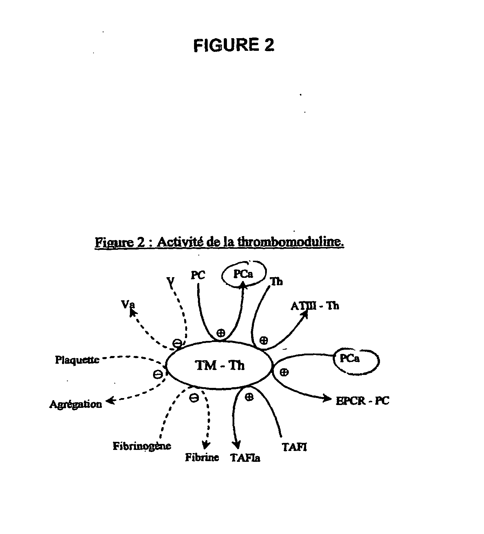

La fonction principale de la TM est de s'opposer à l'action procoagulante de la thrombine, par différents mécanismes. Lors de sa liaison avec la TM, la thrombine (Th) subit une modification conformationnelle. Elle perd ses propriétés coagulantes et son affinité pour les plaquettes et pour le fibrinogène. Toutefois elle peut toujours être inactivée par l'antithrombine III (ATIII). La formation du complexe TM-Th est décuplée en présence de chondroïtine sulfate. Ces interactions permettent à la TM d'éliminer la Th circulante du plasma. Ce complexe TM-Th est capable d'activer la protéine C qui, en présence de protéine S, inhibe les facteurs Va et VIIIa ; cette succession d'interactions et de réaction constitue le système de la protéine C. Le complexe TM-Th joue également un rôle dans l'activation de la protéine C liée au récepteur endothélial à la protéine C (EPCR) ainsi que dans l'activation d'une procarboxypeptidase plasmatique impliquée dans la fibrinolyse : le TAFI (thrombin activatable fibrinolysis inhibitor). Enfin, la TM possède une activité antithrombotique en présence d'ATIII. Un rôle de la TM dans l'inflammation et dans la prolifération / différenciation cellulaire a également été évoqué (Figure 2).

La thrombomoduline agit donc comme un inhibiteur de la coagulation, en particulier en accélérant l'activation de la protéine C en protéine C activée (PCa) qui elle-même, en présence de protéine S cofacteur, inactive les facteurs de la coagulation Va et VIIIa.

La synthèse *in vivo* de thrombomoduline est augmentée par différents facteurs, tels que l'AMPc, les rétinoïdes, les hormones tyroïdiennes, et l'hyperthermie (Conway E.M. et al, 1994, J. Biol. Chem, 269, 22804). D'autres agents tels que les cytokines, IL1, TNF, LPS, et l'homocystéine, ainsi que des microorganismes intracellulaires (cytomégalovirus, Herpès, rickettsies) influencent négativement son expression et son activité, conduisant dès lors à une augmentation transitoire de l'activité procoagulante des cellules endothéliales.

Alors que les taux de TM soluble (TMs) sont élevés chez le nouveau-né, l'âge n'a pas d'influence chez les sujets sains et ce quelle que soit la tranche d'âge considérée entre 20 et 60 ans mais le sexe de l'individu a une influence car on observe des valeurs significativement plus élevées chez l'homme que chez la femme.

Une élévation du taux de TMs associée à une agression de l'endothélium a été décrite dans de nombreuses pathologies telles que la CIVD, le diabète, la leucémie myéloïde chronique, les insuffisances hépatique et rénale, mais également dans les prééclampsies, le purpura thrombotique thrombocytopénique, les rickettsioses, la maladie de Behçet, l'homocystinurie. Des taux diminués ont été observés dans des études sur des malades avec hypertension artérielle pulmonaire et au cours de mélanomes. Enfin des mutations du gène de la TM ont été décrites et associées à une diminution du taux de TMs.

Sur le plan thérapeutique, la perfusion de TM recombinante chez l'animal a montré son efficacité pour prévenir la survenue de thrombose.

Compte tenu de son influence directe sur les étapes de la coagulation, en particulier dans l'activation du système de la protéine C, il est apparu intéressant de pouvoir déterminer l'activité fonctionnelle de la thrombomoduline.

Des tests de détermination d'activité de la thrombomoduline ont d'ores et déjà été proposés dans l'état antérieur de la technique mais ils ne conduisaient pas à la détermination de l'activité de la thrombomoduline dans des systèmes plasmatiques mais uniquement cellulaires. Récemment a été décrite une méthode de dosage en milieu plasmatique par Öhlin A. K. et al (Journal of Thrombosis and Hemostasis, 2005, 3: 976-982). Cependant, cette publication ne propose pas un test direct de la TMs mais nécessite une première étape d'isolement et de séparation par des anticorps monoclonaux dirigés contre la thrombomoduline du plasma. En effet, dans une première étape du test décrit par Öhlin A.K. et al, le plasma testé, éventuellement dilué, est incubé sur une microplaque dont les puits comprennent un anticorps monoclonal contre la thrombomoduline humaine. Après avoir écarté le plasma, les puits des microplaques sont lavés afin de ne conserver que les fragments de thrombomoduline ayant lié l'anticorps. Dans une seconde étape, les puits de la microplaque ainsi préparés sont mis en présence des réactifs nécessaires pour obtenir de la protéine C activée. Après incubation, un substrat chromogénique ou fluorométrique permet la révélation de la protéine C activée.

Les auteurs de la publication concluent qu'il serait important dans le futur, de concevoir un test qui permette de mesurer directement dans le plasma l'activité de la thrombomoduline sans passer par une étape d'isolement et de séparation de la thrombomoduline.

Le dosage de la thrombomoduline plasmatique repose donc actuellement uniquement sur un dosage antigénique de la protéine soluble qui correspond à la protéine membranaire clivée ayant perdu la région cytoplasmique carboxyterminale et transmembranaire hydrophobe. La TMs reste capable de lier la thrombine et d'activer la protéine C, fonctions localisées sur les motifs EGF répétitifs.

Divers tests mettant en oeuvre des réactions immunologiques entre des anticorps antithrombomoduline et la thrombomoduline soluble sont disponibles à ce jour. La société Diagnostica Stago propose par exemple un test ELISA pour le dosage de la thrombomoduline (Asserachrom® Thrombomodulin).

C'est le problème du dosage *in vitro* de l'activité fonctionnelle de la thrombomoduline que résout l'invention en proposant une méthode de mesure de l'activité fonctionnelle de la thrombomoduline, en milieu biologique et en particulier en milieu plasmatique, c'est-à-dire une méthode qui permet le dosage *in vitro* de la thrombomoduline soluble contenue dans le milieu biologique qui la soutient, en particulier dans le plasma, sans séparation de la thrombomoduline des autres constituants du milieu biologique, notamment du plasma, testé.

En d'autres termes, l'invention propose un test de mesure de la thrombomoduline, en milieu biologique et en particulier en milieu plasmatique, en présence des réactifs nécessaires à l'activation du système de la protéine C. En outre, l'invention propose un test qui permet, en milieu biologique et en particulier en milieu plasmatique, de surmonter la difficulté liée à la coagulation de l'échantillon sous l'effet des réactifs nécessaires à l'activation de la protéine C.

La méthode de l'invention et les moyens pour la mettre en oeuvre sont définis pour être utilisables dans le cadre d'examens biomédicaux effectués *in vitro.*

Pour rappel, l'activation de la protéine C en PCa se produit *in vivo* sous l'action du complexe formé à la surface de l'endothélium vasculaire par la thrombomoduline et la thrombine (Facteur IIa). La PCa se complexe ensuite avec la protéine S pour se fixer sur les phospholipides à la surface de plaquettes ou de l'endothélium. Ces complexes PCa-PS-Phospholipides catalysent l'inactivation des facteurs Va et VIIIa, ralentissant ainsi les phénomènes de coagulation.

L'invention concerne donc une méthode de mesure *in vitro* de l'activité fonctionnelle de la thrombomoduline, ladite mesure étant opérée en milieu biologique à partir d'un échantillon préalablement prélevé chez un patient. Selon un mode de réalisation particulier de l'invention, un échantillon permettant la mesure de l'activité thrombomoduline en milieu biologique, est un échantillon de plasma. Selon un autre mode de réalisation particulier de l'invention, l'échantillon permettant la mesure de l'activité thrombomoduline est un échantillon de sang total ou de liquide céphalo-rachidien (LCR) ou de liquide amniotique.

La méthode de dosage *in vitro* selon l'invention comprend la mesure, dans le milieu biologique dé l'échantillon, de l'activation de la protéine C en protéine C activée (PCa), ladite activation étant réalisée sous l'effet de la thrombine, en présence de son cofacteur, la thrombomoduline de l'échantillon. La mesure est faite après addition de protéine C purifiée en excès. D'autres agents nécessaires à l'activation du système de la protéine C sont aussi ajoutés à l'échantillon, ainsi qu'un inhibiteur de la polymérisation de la fibrine.

L'invention a en particulier pour objet une méthode de mesure *in vitro* de l'activité fonctionnelle de la thrombomoduline plasmatique, ladite méthode comprenant le dosage en milieu plasmatique, de l'activation de la protéine C en protéine C activée (PCa) par la thrombine en présence de son cofacteur, ladite thrombomoduline plasmatique. Pour la réalisation de cette mesure, on ajoute, en excès, de la protéine C purifiée, dite protéine C exogène purifiée, au plasma testé, ainsi que des agents nécessaires à l'activation du système de la protéine C et un inhibiteur de la polymérisation de la fibrine.

Alternativement à l'addition de protéine C purifiée, on peut ajouter du TAFI (Carboxipeptidase U plasmatique dont l'activation catalysée par la thrombine, est accrue pour la thrombomoduline) pour mesurer son activation en TAFI activé (TAFIa).

La mesure de l'activité est dite une mesure de l'activité fonctionnelle de la thrombomoduline, car elle permet de mesurer l'activité de la thrombomoduline en milieu biologique, en particulier en milieu plasmatique en ajoutant au milieu, notamment au plasma testé, de la protéine C en excès et les agents nécessaires à l'activation du système de la protéine C. On observera que l'excès de protéine C utilisée est suffisant pour s'affranchir, lors de la mesure, de tout déficit quantitatif ou qualitatif relatif à la présence dans le milieu testé, de protéine C endogène. En d'autres termes, par « excès de protéine C exogène » on entend donc pour la réalisation de l'invention que la protéine C exogène est ajoutée en quantité telle que la mesure de son activation en PCa est indépendante de la teneur en protéine C de l'échantillon.

Selon un mode de réalisation particulier de l'invention, le dosage effectué en milieu plasmatique est réalisé sur un échantillon de plasma obtenu à partir d'un échantillon biologique préalablement prélevé chez un patient.

La thrombomoduline plasmatique que l'on mesure selon l'invention est la protéine soluble (thrombomoduline soluble ou TMs dite aussi thrombomoduline plasmatique TMp) qui correspond à la thrombomoduline membranaire clivée et donc séparée de ses régions cytoplasmique carboxyterminale et transmembranaire hydrophobe. La thrombomoduline soluble reste en effet capable de lier la thrombine et d'activer la protéine C en PCa puisque cette fonction d'activation est localisée sur les motifs EGF répétés de la protéine.

En revanche, lorsque l'activité de la thrombomoduline est mesurée dans un échantillon de LCR ou de liquide amniotique, la thrombomoduline est sous sa forme complète (non tronquée).

Les agents nécessaires à l'activation du système de la protéine C que l'on apporte à l'échantillon de plasma testé sont principalement pour les différents modes de réalisation de l'invention, la thrombine (en particulier l'a-thrombine humaine purifiée) et des ions calcium.

Les ions calcium sont par exemple apportés sous forme de CaCl2.

En outre, la méthode de mesure de l'activité de la thrombomoduline plasmatique selon l'invention comporte l'apport au niveau de l'échantillon testé, d'un inhibiteur entrant en compétition avec la thrombine et bloquant ainsi la transformation du fibrinogène en fibrine.

Une méthode de mesure *in vitro* de l'activité fonctionnelle de la thrombomoduline plasmatique selon l'invention comprend :
- la mise en contact d'un échantillon de plasma testé pour l'activité de la thrombomoduline, avec de la protéine C exogène purifiée, de la thrombine, des ions calcium et un inhibiteur de la polymérisation de la fibrine;
- l'incubation du milieu obtenu pour permettre la production de la protéine C activée ;
- le dosage fonctionnel de la protéine C activée.

Dans un mode de réalisation particulier de la méthode de mesure selon l'invention, la quantité de protéine C activée formée est dosée par son activité sur un substrat. Ce substrat est en particulier un substrat enzymatique, naturel ou synthétique; par exemple il s'agit d'un substrat chromogénique ou fluorométrique.

Un substrat approprié pour doser l'activité de la PCa peut être un substrat protéique, par exemple un peptide.

Dans un mode de réalisation particulier de l'invention, le substrat enzymatique de la protéine C activée mesurée est un substrat permettant de doser l'activité amidolytique de la PCa. En particulier, il s'agit d'un substrat chromogénique ou fluorométrique.

Au titre des substrats synthétiques utilisables dans le cadre de l'invention, on citera les substrats oligopeptidiques synthétiques. A titre d'exemple, on mentionne le substrat synthétique CBS 42.46 de la société Diagnostica Stago (oligopeptide : THC-Pro-Arg-pNa). Un autre exemple de substrat chromogénique est le substrat S-2366 (oligopeptide pyroGlu-Pro-Arg-pNa) ou S-2238 (oligopeptide H-D-Phe-Pip-Arg-pNa).

Dans le cadre de l'invention, différents inhibiteurs de polymérisation de la fibrine peuvent être choisis. On cite à titre d'exemple, les inhibiteurs peptidiques synthétiques, par exemple l'inhibiteur oligopeptidique H-Gly-Pro-Arg-Pro-OH.AcOH (GPRP.AcOH) (Pefabloc® FG de la société Pentapharm).

Dans un mode de réalisation particulier de l'invention, la méthode de mesure de l'activité fonctionnelle de la thrombomoduline plasmatique comprend les étapes suivantes :
a) la mise en contact d'un échantillon de plasma avec un réactif 1 comprenant la thrombine, contenue dans un milieu permettant l'activation du système de la protéine C, en particulier comprenant des ions calcium, le réactif 1 comprenant en outre un inhibiteur de la polymérisation de la fibrine ;
b) l'incubation du milieu constitué à l'étape a) avec un réactif 2 comprenant de la protéine C purifiée, pendant un temps suffisant pour permettre son activation en PCa par la thrombine complexée à son cofacteur, la thrombomoduline contenue dans l'échantillon de plasma ;
c) la mise en contact du milieu constitué à l'étape b) comprenant la protéine C activée, avec un substrat enzymatique de la PCa ;
d) la quantification de la transformation du substrat de la PCa.

Pour la réalisation de l'invention, la protéine C utilisée est une protéine C purifiée, par exemple la protéine C isolée selon la méthode décrite par Öhlin A.K. et al, J. Biol. Chem., 1988, 263 : 19240-8. Selon un mode de réalisation particulier de l'invention, la protéine C utilisée est la protéine C humaine purifiée. La protéine C utilisée est apportée à une concentration comprise dans l'intervalle 0,5 µM/l et 2,5 µM/l, voire 0,1 à 10 µM/l.

La protéine C est dite purifiée pour exprimer qu'elle est substantiellement dépourvue d'autres composants protéiques et de contaminants, indépendamment de toute référence à son mode de préparation.

La protéine C apportée peut être une protéine recombinante.

La protéine C est aussi disponible sous forme purifiée et active pour la réalisation de la mesure selon l'invention, auprès des sociétés Diagnostica Stago, Enzyme Research Laboratories, American Diagnostica.

Dans le cadre de la méthode de l'invention, la mise en contact de la thrombine apportée avec la thrombomoduline présente dans le plasma testé, conduit à la formation du complexe thrombine / thrombomoduline.

Le réactif apportant la thrombine et les ions calcium peut être constitué par la thrombine diluée dans un tampon comprenant les ions calcium. L'inhibiteur de la polymérisation de la fibrine peut également être ajouté au tampon. D'autres constituants habituellement contenus dans ce type de tampon peuvent en outre être ajoutés.

La thrombine utilisée, est avantageusement d'origine humaine. En particulier, il s'agit de thrombine dont l'activité, exprimée en unités NIH, est dans l'intervalle de 5 à 20 NIH, avantageusement 10NIH dans le réactif qui est utilisé pour préparer le milieu de réaction.

Le réactif ainsi constitué permet l'interaction de la thrombine apportée avec la thrombomoduline de l'échantillon de plasma et l'activation de la protéine C.

Parmi les autres composants du réactif constitué, on cite par exemple un inhibiteur de l'héparine ou d'un autre anticoagulant, utilisé dans la mesure où l'échantillon à tester a été préalablement prélevé chez un patient traité avec de l'héparine ou avec un autre anticoagulant et est donc caractéristique d'un patient traité avec de l'héparine ou un autre anticoagulant.

Si toutefois l'échantillon provient d'un patient traité avec un anticoagulant de la famille des anti-vitamine K, on n'ajoute pas d'inhibiteur de cet anticoagulant, dans la mesure où aucun inhibiteur n'est à ce jour disponible.

Alternativement à la dilution de l'échantillon en tampon Owren Koller, on propose de réaliser la dilution dans un plasma normal selon les critères habituels de sélection connus de l'homme du métier, ledit plasma étant rendu déficient en thrombomoduline. On ajoute alors à ce plasma la thrombine exogène ainsi que la protéine C purifiée et l'inhibiteur de la polymérisation de la fibrine et comme indiqué ci-dessus, l'inhibiteur de l'héparine lorsque l'échantillon biologique provient d'un patient traité avec de l'héparine, ou un autre inhibiteur s'il s'agit d'un anticoagulant différent. L'apport de thrombine est réalisé par le plasma.

On entend par plasma déficient thrombomoduline un plasma qui comprend moins de 1 % de cette protéine.

Un plasma déficient en thrombomoduline peut être préparé par toute méthode en soi connue.

On peut par exemple utiliser la technique d'immunoadsorption en mettant en oeuvre des anticorps dirigés contre cette protéine, par exemple des anticorps immobilisés sur un support.

Le plasma à traiter est citraté.

La protéine C purifiée, est apportée dans des conditions permettant sa réaction avec le complexe thrombine/thrombomoduline conduisant à l'activation de cette dernière sous la forme d'une protéine C activée (PCa). C'est sous cette forme de PCa, que la protéine C, interagissant avec les ions calcium ainsi que des phospholipides et son cofacteur la protéine S, est capable d'interagir avec ses substrats et notamment dans le processus de coagulation, les Facteurs Va et VIIIa (FVa, FVIIIa). On peut ainsi doser l'inhibition du facteur Va pour mesurer l'activité de la thrombomoduline.

La mesure de l'activité fonctionnelle de la protéine C activée comprend la réaction du réactif obtenu, avec un substrat enzymatique de la protéine C activée.

Ce substrat a été décrit plus haut et on rappelle qu'il peut être un substrat naturel ou synthétique. Lorsqu'il s'agit d'un substrat naturel, il peut s'agir d'une protéine plasmatique de la coagulation, telle que le FVa ou le FVIIIa. Alternativement, le substrat mesuré, révélateur de l'activité fonctionnelle de la thrombomoduline peut être le TAFI activé (TAFIa) ou un substrat du TAFI activé. Lorsque le substrat enzymatique de la PCa est de nature synthétique, il peut s'agir par exemple d'un substrat peptidique. Avantageusement, le substrat en question est un substrat chromogénique ou fluorométrique.

En particulier, il peut s'agir d'un substrat permettant de doser l'activité amidolytique de la PCa. Un tel substrat est par exemple le substrat oligopeptidique synthétique CBS42.46 de la société Diagnostica Stago. D'autres substrats ont été cités précédemment à titre d'exemple.

L'étape de quantification de l'activité fonctionnelle de la thrombomoduline est basée sur la quantification de la transformation du substrat de la PCa.

Cette quantification peut être réalisée en mesurant la densité optique du mélange réactionnel dans une fenêtre de lecture déterminée en fonction du substrat choisi.

Dans un mode de réalisation particulier de l'invention, le plasma obtenu à partir de l'échantillon biologique prélevé chez le patient est dilué. Par exemple, le plasma est dilué moins de vingt fois, par exemple moins de dix fois et notamment est dilué au 1/3 ou au 1/4.

La dilution réalisée peut être décidée en fonction de la sensibilité du substrat : plus le substrat est sensible, plus la dilution peut être forte.

Dans un mode de réalisation particulier de l'invention, l'inhibiteur de la polymérisation de la fibrine est un inhibiteur polypeptidique. En particulier, il peut s'agir de l'oligopeptide H-Gly-Pro-Arg-Pro-OH.AcOH GPRP.AcOH /Pefabloc® FG de la société Pentapharm). Cet inhibiteur peut être utilisé dans la réaction de dosage, à une concentration variant de 1 à 15 g/l, choisie selon la dilution du plasma échantillon. Par exemple, lorsque le plasma est dilué au 1/3, on utilisera avantageusement 10 g/l de cet inhibiteur.

Dans un autre mode de réalisation de l'invention, l'inhibiteur de la polymérisation de la fibrine est un anticorps anti-fibrinogène tel que décrit ci-dessus.

Dans un mode de réalisation particulier de l'invention, la mesure de l'activité fonctionnelle de la thrombomoduline comprend l'utilisation du tampon thrombine ayant la constitution suivante :
NaCl (0,15 mmol/l), Tris (20 mmol/l), CaCl2 (2,5 mmol/l), BSA (5mg/ml) et,
lorsque l'échantillon de plasma provient d'un patient sous héparine, un inhibiteur de l'héparine, par exemple le polybrène (10g/l).

Tout autre tampon adapté pour la dilution de la thrombine peut être utilisé.

Lorsque le substrat enzymatique de la protéine C activée est le CBS42.46, ou un autre substrat enzymatique notamment un substrat synthétique, la lecture de la densité optique peut être faite dans une fenêtre de lecture allant de 6 à 500 secondes, à une longueur d'onde de 405 nm.

Alternativement, la densité optique peut être lue dans une fenêtre allant de 6 à 200 secondes, à une longueur d'onde de 405nm.

La concentration de substrat, notamment de CBS42.46 est par exemple de 2,5µM/ml dans le réactif à apporter dans le milieu réactionnel.

L'étape d'incubation du plasma mis en contact avec la thrombine en présence d'ions calcium, avec la protéine C peut être réalisée pendant une durée d'environ 1 à 120 minutes, par exemple d'environ 600 secondes.

Ainsi, dans un mode de réalisation particulier de l'invention, la méthode de mesure de l'activité fonctionnelle de la PCa est mise en oeuvre dans les conditions suivantes :
- pour l'étape a), on met en contact 70 µl d'échantillon de plasma dilué au 1/3, avec 40 µl de thrombine 10 NIH diluée dans un tampon contenant NaCl (0,15 mmol/l), Tris (20 mmol/l), CaCl2 (2,5 mmol/l), BSA (5mg/ml) et en présence d'inhibiteur de la polymérisation de la fibrine, par exemple *GPRP.AcOH* (10g/l) et, lorsque l'échantillon de plasma provient d'un patient sous héparine, un inhibiteur de l'héparine, par exemple le polybrène (10g/l) ;
- pour l'étape b) 30 µl de protéine C purifiée sont incubés pendant 600s avec le milieu constitué à l'étape a) ;
- pour l'étape c) 110 µl de substrat enzymatique de la protéine C activée, par exemple de CBS 42.46 à 2,5µM/ml (dans le réactif), et la quantité de substrat enzymatique transformé est déterminée par lecture optique dans une zone de 6 à 500 s, à une longueur d'onde de 405 nm.

Dans un mode de réalisation particulier de l'invention, la mesure de l'activité fonctionnelle de la thrombomoduline plasmatique est faite parallèlement à la mesure de la même activité d'un contrôle interne. Ce contrôle peut être obtenu en utilisant un plasma standard traité comme l'échantillon.

Les quantités et/ou concentrations de réactifs à apporter dans le milieu réactionnel pour la mise en oeuvre de la méthode, telles que les quantités et/ou concentrations de plasma, de thrombine, de protéine C purifiée, d'inhibiteur de polymérisation de la fibrine, et de substrat peuvent varier par rapport aux données décrites dans la présente demande, notamment en fonction de la concentration de chaque réactif ou de son activité. De préférence les concentrations finales respectives des différents réactifs de la réaction sont sensiblement conservées dans le milieu réactionnel.

Par concentration finale on entend la concentration des réactifs dans le milieu où ils agissent quand on les y a introduits.

L'homme du métier est en mesure d'adapter les concentrations et activités, en fonction des réactifs choisis. A titre illustratif, on notera que les concentrations ou les activités des réactifs, peuvent varier à hauteur de plus ou moins 50%, par exemple à hauteur de plus ou moins 20%, ou de plus ou moins 10%, par rapport aux indications contenues dans la présente demande. Cette variation peut concerner chaque réactif, indépendamment des autres réactifs.

Pour quantifier l'activité fonctionnelle de la thrombomoduline, selon un mode de réalisation particulier, l'invention propose de comparer le résultat obtenu de la réaction, par exemple la valeur lue de la densité optique, à des valeurs établies sur une courbe de calibration. Cette courbe de calibration par exemple est obtenue en mesurant l'activité fonctionnelle de la thrombomoduline au moyen
- d'un échantillon de plasma standard dilué en tampon Owren Koller, par exemple un plasma qui comprend des quantités statistiquement habituelles, dites normales, des facteurs de la coagulation connus dans les voies endogène et exogène de la coagulation. Il peut être obtenu à partir d'échantillons normaux de plasma, soit d'échantillons normaux utilisés en pool, soit d'échantillons testés individuellement pour ensuite déterminer une valeur moyenne des valeurs individuelles obtenues de l'activité de la thrombomoduline. Les valeurs statistiquement habituelles des facteurs de coagulation sont situées dans des intervalles donnés dans les manuels connus de l'homme du métier. A titre d'exemple, on considérera comme plasma normal, un plasma donnant des resultats qualifiés de normaux dans des tests du fibrinogène, du temps de céphaline avec activateur (TCA), du temps de thrombine (TB), de la PC et du facteur V. A cet égard, on peut aussi se reporter aux normes définies dans l'ouvrage : « How to define and determine référence intervals in the clinical laboratory : Approved Guidelines - Second Edition. NCCLS document C28-A2 (ISBN 1-56238-406-6) NCCLS-USA 2000. » ou,
- d'un plasma déplété en thrombomoduline auquel est ajoutée une quantité croissante de TM purifiée. II peut être obtenu par toute méthode en soi connue, par exemple par immunoadsorption au moyen d'anticorps antithrombomoduline, pour réaliser la déplétion en TM avant l'addition de TM purifiée.

On peut ainsi former une gamme de dilution, de plasmas normaux, d'un pool de plasmas normaux ou de plasmas rendus déficients en TM auxquels on ajoute des quantités croissantes de TM.

La thrombomoduline ajoutée aux échantillons de plasma pour établir la courbe de calibration peut être complète ou soluble.

II peut s'agir aussi d'une molécule recombinante tronquée et soluble de thrombomoduline, comme la solulin® commercialisée par la société Berlex Biosciences, San Francisco USA. Cette molécule peut être utilisée comme standard pour la réalisation d'un contrôle interne ou pour la réalisation d'une courbe de calibration.

Suivant les caractéristiques des patients susceptibles d'être soumis au test de mesure de l'activité de la TM, on pourra ajuster le choix des échantillons de plasmas utilisés pour obtenir les valeurs des contrôles.

Ainsi, les échantillons de plasmas sélectionnés comme contrôles seront prélevés avantageusement chez des individus présentant des caractéristiques voisines de celles des patients testés, pour ce qui concerne la tranche d'âge, le sexe, l'indice de masse corporelle, et le cas échéant, la prévalence de l'usage du tabac ou d'alcool.

L'invention a également pour objet un kit pour la mesure de l'activité de la thrombomoduline plasmatique, en milieu plasmatique, comprenant :
- un réactif 1 comprenant la thrombine diluée dans un tampon comprenant un inhibiteur de la polymérisation de *la fibrine* et comprenant des ions calcium et le cas échéant un inhibiteur de l'héparine ;
- un réactif 2 comprenant la protéine C purifiée ; ou alternativement du TAFI (Thrombin Activatable Fibrinolysis Inhibitor)
- un réactif 3 comprenant un substrat enzymatique de la protéine C activée, ou un substrat spécifique du TAFIA.

Le dosage de l'activité du TAFI consistant à mesurer le TAFIa (TAFI activé) a par exemple été décrit dans le brevet WO 03/004516, au moyen de substrats chromogènes du TAFIa dérivés de composés arazoformyl.

Selon un mode de réalisation particulier de l'invention, le kit est caractérisé en ce que le réactif 1 comprend essentiellement les composés suivants : NaCl (0,15 mmol/l), Tris (20 mmol/l), CaCl2 (2,5 mmol/l), BSA (5mg/ml) et pour des échantillons de plasma *de malades* traités par l'héparine ou un autre anticoagulant, un inhibiteur de l'héparine ou dudit anticoagulant, par exemple le polybrène (10g/l) et un inhibiteur de la polymérisation de la fibrine.

Selon un mode de réalisation particulier de l'invention, l'inhibiteur de la polymérisation *de la fibrine* est le *GPRP.AcOH* utilisé à une concentration de 1 à 15 g/l. La concentration est déterminée en fonction du degré de dilution du plasma testé. Par exemple, lorsque le plasma est dilué au tiers, on utilise 10 g/l du *GPRP.AcOH.* Les propriétés et conditions d'utilisation de cet inhibiteur ont été décrites plus haut.

L'invention a aussi pour objet un kit tel que défini ci-dessus, dans lequel l'inhibiteur de la polymérisation de la fibrine est un anticorps anti-fibrinogène.

Dans un mode de réalisation particulier, le kit comprend en outre un contrôle interne défini comme indiqué dans la présente demande.

Selon un mode de réalisation particulier, le kit peut encore comprendre une courbe de calibration de l'activité fonctionnelle de la thrombomoduline, ou les moyens propres à la réalisation d'une telle courbe.

L'invention a également pour objet une méthode de détection *in vitro* d'une anomalie de la coagulation, comprenant la mesure de l'activité fonctionnelle de la thrombomoduline en milieu plasmatique, telle que décrite dans la présente demande. Il peut s'agir de détecter une augmentation du taux de l'activité fonctionnelle de la thrombomoduline ou une diminution de ce taux.

Chez un sujet adulte, on considère qu'une variation (élévation ou diminution) anormale du taux de thrombomoduline est un marqueur de risque thrombotique ou de lésion(s) endothéliale(s).

Selon un mode de réalisation particulier de cette méthode, on compare l'activité fonctionnelle mesurée de la thrombomoduline en milieu plasmatique à une valeur de cette activité, mesurée sur un plasma standard.

Dans un mode de réalisation particulier de l'invention, la valeur ainsi obtenue dite valeur standard ou normale est obtenue après dosage de l'activité fonctionnelle de la thrombomoduline sur un pool d'échantillons de plasmas standards.

Selon un autre mode de réalisation particulier de l'invention, ladite valeur standard est établie à partir de valeurs de l'activité fonctionnelle de la thrombomoduline mesurées sur des échantillons de plasmas normaux (standards) dosés individuellement.

Les moyens, méthodes et kits décrits dans la présente demande sont utilisables pour la détection d'une augmentation du taux de thrombomoduline, éventuellement associée à des symptômes cliniques. Ainsi, une augmentation du taux de thrombomoduline peut être associée à des symptômes de coagulation intravasculaire disséminés (CIVD), de diabète, de leucémie myéloïde chronique, d'insuffisance hépatique et rénale, de prééc/ampsie, de purpura thrombotique thrombocytopénique, de rickettsioses, de maladie de *Behçet,* d'homocystinurie, de fausse couche.

Selon un autre mode de réalisation de l'invention, les moyens, méthodes et kits, sont utilisés pour la détection d'une diminution du taux de thrombomoduline éventuellement associée à des symptômes cliniques. Ces symptômes peuvent être des symptômes d'hypertension artérielle pulmonaire ou à des mélanomes ou autre.

Dans un autre mode de réalisation de l'invention, les moyens, méthodes et kits décrits, sont utilisés pour la détection d'une diminution du taux de thrombomoduline associée à une mutation du gène de la thrombomoduline.

D'autres caractéristiques et avantages de l'invention apparaissent dans les exemples qui suivent en particulier pour illustrer les variations du taux de thrombomoduline liées à des états pathologiques et dans les figures.
Figure 1 : Schéma de l'organisation structurelle de la thrombomoduline : EGF=Epidermal growth factor (EGF-like). La séquence de la thromboduline est décrite dans l'article de Weiler H. et Isermann B.H. de 2003 (Journal of Thrombosis and Haemostasis, vol. 1, pages 1515-1524).
Figure 2 : Schéma de l'activité de la thrombomoduline : PC : protéine C ; PCa : protéine C activée ; Th : thrombine ; ATIII-Th : complexe anti-thrombine III-thrombine ; EPCR-PC : Récepteur Endothélial de la Protéine C - Protéine C ; TAFI : Thrombin Activatable Fibrinolisis inhibitor ; TAFIa : TAFI activé ; TM : Thrombomoduline ; V : Factor V ; Va : Factor V activé.
Figure 3 : Courbe illustrant la spécificité du dosage. (Def TM : déficient en TM).
Figure 4 : Détection de plasmas pathologiques et de plasmas normaux.
Figure 5 : Courbe de calibration de la mesure (DDO) de l'activité de la thrombomoduline en fonction de sa concentration.
Figure 6 : Mesure de la sensibilité de l'activité de la thrombomoduline, en fonction du taux de protéine C de l'échantillon :
Figure 7 : Sensibilité au taux de facteur II de l'échantillon.
Figure 8 : Sensiblité au taux de TAFI de l'échantillon.
Figure 9 : Estimation de la stabilité.
Figure 10 : Mesure de la concentration en phospholipides et de l'activité TMa sur des échantillons de plasmas de femmes ayant une grossesse normale ou sujette à complications.

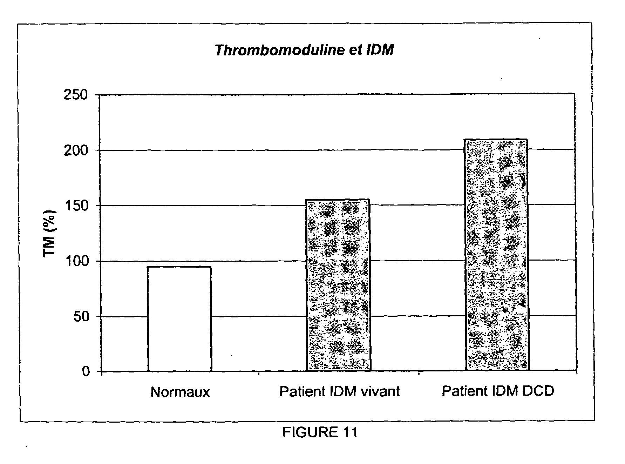

Figure 11 : activité de la thrombomoduline corrélée avec l'infarctus du myocarde (IDM)

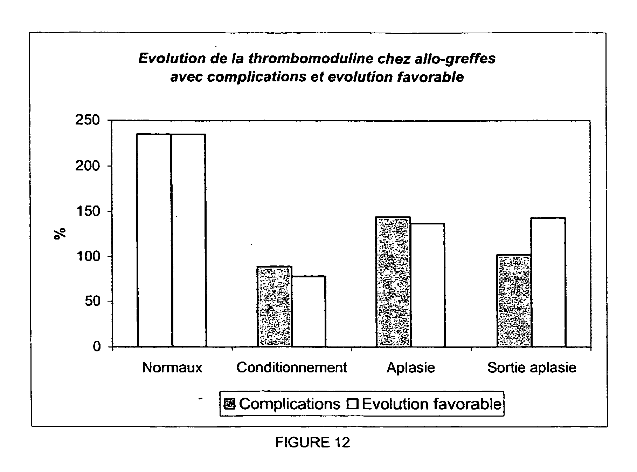

Figure 12 : évolution de la thrombomoduline chez des patients ayant subi une allo-greffe. L'activité TMa a été testée sur des échantillons de patients dont l'état évoluait avec des complications, et sur des échantillons de patients dont l'état évoluait favorablement

Les conditions de l'obtention de ces résultats sont décrites dans les exemples.

### EXEMPLES

### I Méthode de dosage

Le dosage de l'activité fonctionnelle de la thrombomoduline a été réalisé comme suit, sur un échantillon de plasma de 70 µl dilué au 1/3.

### A) Réactifs à apporter au milieu réactionnel

- 40 µl de Thrombine 10 NIH (soit 1,6 NIH en final dans le milieu) en tampon thrombine constitué par :
   0.15 mol/lNacl
   20 mmol/l Tris
   2.5 mmol/l CaCl2
   5 mg/ml BSA
   10 g/l GPRP.AcOH (inhibiteur de polymérisation de la fibrine) 2ml/l polybrène 10g/l
- 30 µl de protéine C purifiée à 100 µg/ml reprise par 1 ml d'eau distillée
- 110 µl de substrat synthétique de la PCa (CBS42.46 de la société Diagnostica Stago à 2,5µM/ml) repris par 6 ml d'eau distillée / Hirudine à 20 ATU) (soit en concentration finale dans le milieu 1,10µM/ml)

### B) Activation de la protéine C et dosage de la PCa

L'échantillon de plasma a été mis en contact avec la thrombine dans le tampon thrombine et incubé à 37°C avec la protéine C, pendant une durée d'environ 600 secondes, pour activer la protéine C.

Le milieu obtenu a été mis en contact avec le substrat synthétique.

L'activité amidolytique de la PCa a ensuite été déterminée à 37°C par lecture de densité optique dans une zone de lecture de 6 à 500 sec, à 405 nm.

### C) Contrôle de la spécificité de la mesure

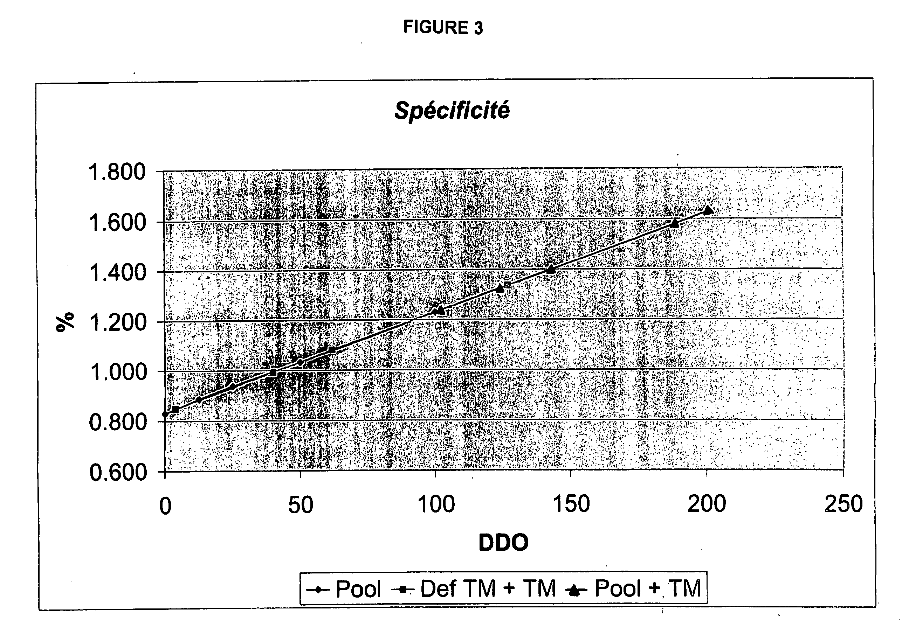

La spécificité de la mesure de la densité optique par rapport à l'activité de la PCa a été vérifiée en comparant l'activité obtenue avec un pool de plasmas normaux et avec un plasma déficient en TM auquel on a ajouté la TM purifiée (de chez American Diagnostica ou Haematologic Technologies INC par exemple). Les résultats sont présentés à la figure 3 et confirment la spécificité du dosage.

### D) Contrôle de la répétabilité

Les mesures de répétabilité ont été opérées et les résultats sont rapportés aux tableaux 1-3.

### E) Valeurs d'activité de la PCa

43 plasmas normaux ont été dosés pour l'activité thrombomoduline, suivant la méthode de l'invention. Les mesures de densité optique et les pourcentages d'activité TM *correspondants* sont rapportés au Tableau 4. Ces mesures peuvent être considérées comme indicatrices des valeurs normales d'activité fonctionnelle de la thrombomoduline.

**Tableau 4**

| Plasmas normaux | | |
|---|---|---|
| | *Normaux (n=43)* | |
| | DDO | % |
| Moyenne | 1.281 | 106 |
| Médiane | 1.291 | 109 |
| Min | 1.122 | 65 |
| Max | 1.451 | 151 |

On a considéré que 100% d'activité fonctionnelle TM est défini arbitrairement comme le taux de TM qui résulte en la formation de 0,03 µmol/l de PCa quand les concentrations en thrombine et en PC sont respectivement de 10 NIH et 100 µg/ml en présence de 2,5 mmol/l d'ions Ca²⁺.

Par ailleurs, des plasmas obtenus à partir d'échantillons biologiques prélevés chez des patients affectés par des pathologies identifiées, ont été également dosés pour l'activité fonctionnelle de la TM, selon la méthode de l'invention. Les mesures ont été réalisées dans un analyseur STA-R (Diagnostica Stago, France) en utilisant le test chromogénique avec le substrat synthétique évoqué ci-dessus. Les valeurs de Cts intra et inter essais sont respectivement <4% et <5%.

La réponse au test est linéaire entre 0 et 200%. Les résultats en pourcentage d'activité TM sont les suivants: diabètes (n=15) : 130% ±28 ; cancers (n=10) : 155 ± 60 ; sepsis (n=12) : 173% ±56 ; mutiple trauma (n=20) : 185% ± 62.

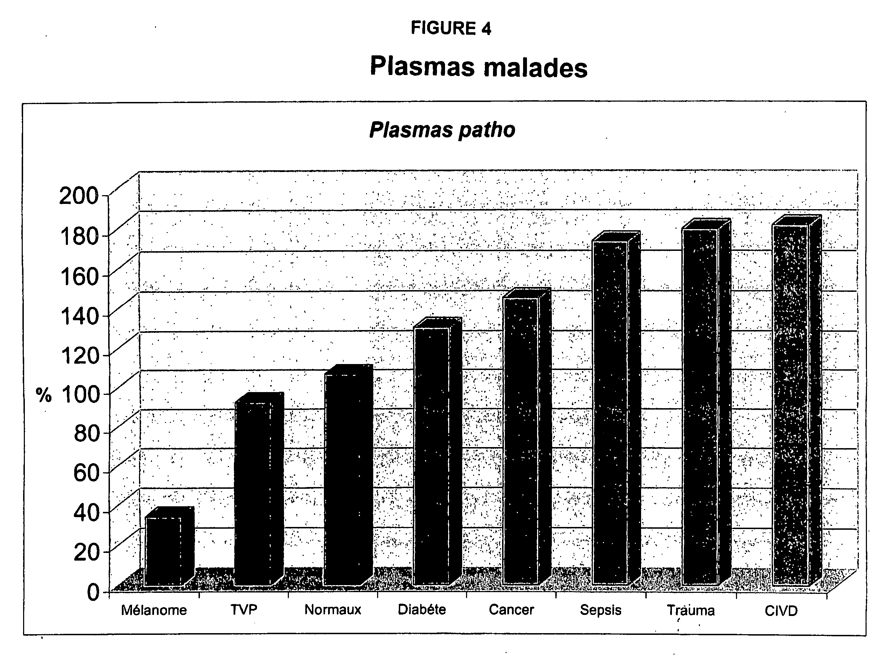

Ces résultats sont illustrés à la figure 4.

### II Exemple de préparation d'une courbe de calibration

Pour établir la courbe de calibration, donnant l'activité fonctionnelle de la thrombomoduline, on a utilisé
- soit la dilution d'un pool de plasmas normaux en tampon Owren Koller ou dans un déficients en thrombomoduline.
- soit la surcharge d'un plasma déficient en thrombomoduline par de la thrombomoduline purifiée.
- soit en définissant arbitrairement le taux de 100 % d'activité thrombomoduline comme étant de taux de thrombomoduline qui résulte en la formation de 0,03 µml/l de PCa quand la concentration en thrombine est de 10 NIH et la concentration en PC est de 100 µg/ml, en présence de 2,5 mmol/l de Ca²⁺.

Chaque dilution de plasmas est testée au 1/3 dans la configuration.

On a déterminé la concentration en TM ajoutée au pool de plasmas correspondant à une activité de 200%, 150%, 100%, 50%, 25%, 12,5% et 0% de la thrombomoduline suivant la règle ci-dessus définie.

L'activité de la thrombomoduline a été déterminée sur un substrat synthétique de la PCa, le CBS 42.46. On a mesuré l'activité amidolytique de la PCa par la libération de paranitroaniline (de couleur jaune) à une longueur d'onde de 405 nm.

On a déterminé la densité optique, qui correspond à chaque valeur (en pourcentage) d'activité de la thrombomoduline, dans une fenêtre de lecture allant de 6 à 500 secondes à une longueur d'onde de 405 nm.

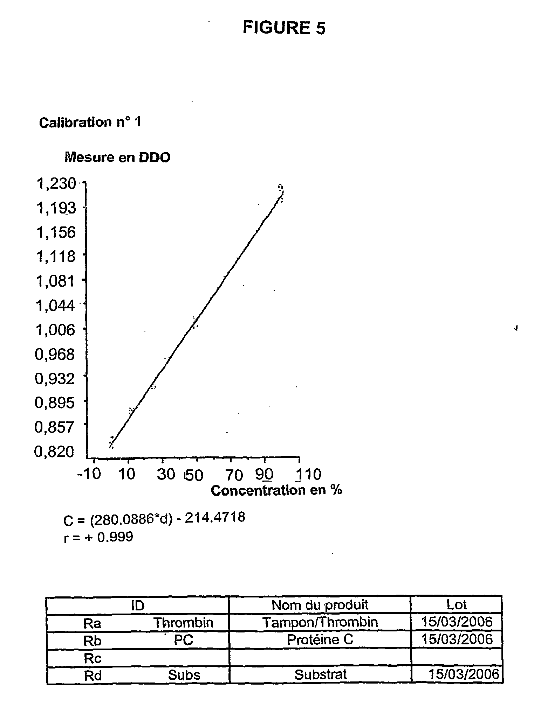

Une courbe de calibration est présentée à la figure 5.

### III Evaluation de la sensibilité du dosage à différentes protéines plasmatiques de la coagulation, ou à des agents interférant avec la coagulation.

### A) Sensibilité à l'héparine

La sensibilité a été évaluée en ajoutant au plasma dosé dans les conditions de dosage décrites ci-dessus, des concentrations croissantes de calciparine®.

L'influence de la calciparine est décrite dans le tableau 6 suivant et s'avère peu déterminante sur l'activité fonctionnelle de la TM.

**Tableau 6**

| Sensibilité à l'héparine | | | |
|---|---|---|---|
| | DDO | % | Recouvrement % |
| Calciparine 0 | 1.183 | 119 | 100 |
| Calciparine 0.4 | 1.135 | 107 | 90 |
| Calciparine 0.8 | 1.159 | 113 | 95 |
| Calciparine 1.0 | 1.199 | 123 | 103 |
| Calciparine 1.4 | 1.239 | 133 | 111 |
| Calciparine 1.8 | 1.271 | 141 | 118 |
| Calciparine 2.0 | 1.214 | 127 | 106 |

### B) Sensibilité au taux de protéine C contenue dans l'échantillon de plasma

Des plasmas comprenant une concentration variable déterminée en protéine C ont été dosés selon la méthode décrite ci-dessus.

Il s'agit de :
- Plasma déficient en protéine C (colonne 0) ;
- Plasmas d'un pool rendus plus ou moins déficients en protéine C (colonnes 12.5-50) ;
- Plasma d'un pool normal (pool GK (Georges King)) (colonne 100) ;

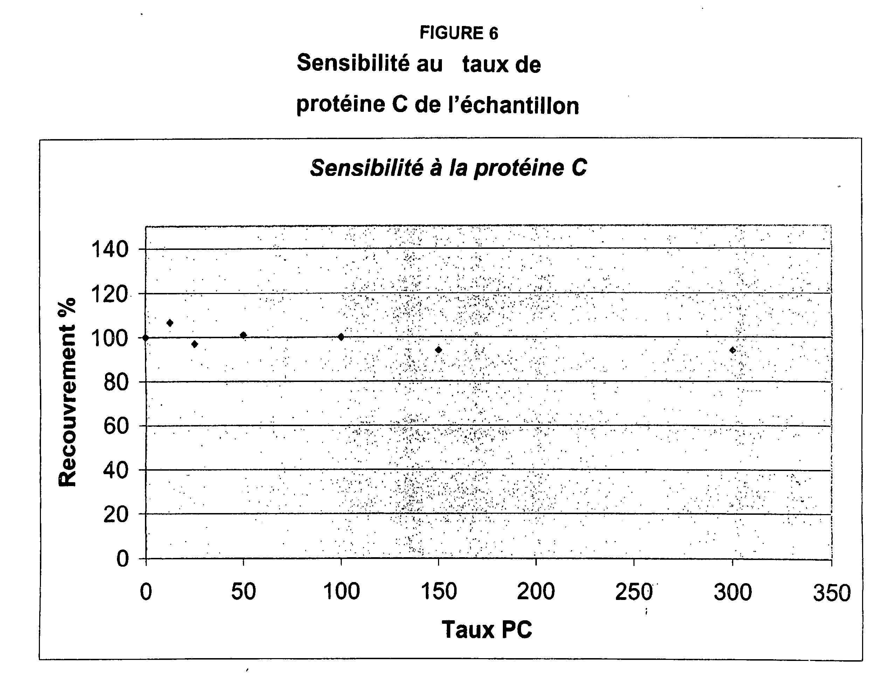

Les résultats (Tableau 7 et figure 6) montrent que le taux de PC de l'échantillon influence peu la valeur de l'activité fonctionnelle de la TM.

**Tableau 7**

| Sensibilité au taux de protéine C de l'échantillon (1) | | | | | | | |
|---|---|---|---|---|---|---|---|
| Taux de PC (%) | 0 | 12.5 | 25 | 50 | 100 | 150 | 300 |
| Tx théorique (%) | 110 | 103 | 104 | 106 | 102 | 102 | 102 |
| Tx mesuré (%) | 110 | 110 | 101 | 107 | 102 | 96 | 96 |
| Recouvrement | 100 | 107 | 97 | 101 | 100 | 94 | 94 |

| | | | | | | | |
|---|---|---|---|---|---|---|---|
| 0= Déf PC Clinisys 12.5-50= Pol/Déf PC 100 = Pool GK 150-300= Pool/PC purifiée | | | | | | | |

### C) Sensibilité au taux de Facteur II contenu dans l'échantillon de plasma

Des plasmas comprenant une concentration variable déterminée en Facteur II ont été dosés selon la méthode décrite ci-dessus.

Il s'agit de :
- Plasma déficient en Facteur II (colonne 0) ;
- Plasmas d'un pool rendus plus ou moins déficients en Facteur II (colonnes 25-87,5) ;
- Plasma d'un pool normal (pool GK) (colonne 100) ;

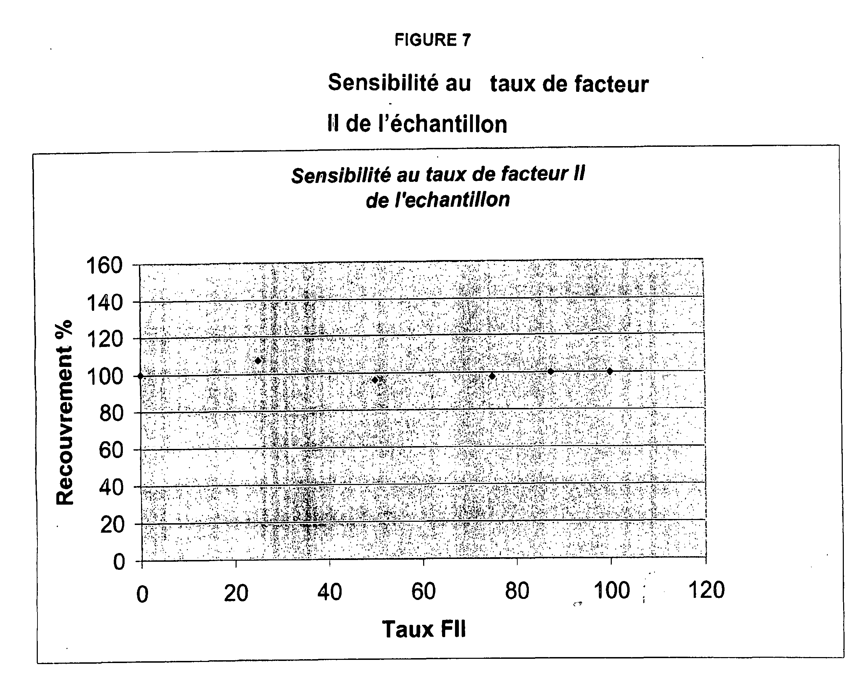

Les résultats (Tableau 8 et figure 7) montrent que le taux de FII de l'échantillon influence peu la valeur de l'activité fonctionnelle de la TM.

**Tableau 8**

| | | | | | | |
|---|---|---|---|---|---|---|
| Taux de FII (%) | 100 | 87.5 | 75 | 50 | 25 | 0 |
| Taux théorique (%) | 100 | 95 | 90 | 79 | 69 | 58 |
| Taux mesuré (%) | 100 | 95 | 88 | 76 | 74 | 58 |
| Recouvrement (%) | 100 | 100 | 98 | 96 | 107 | 100 |

| | | | | | | |
|---|---|---|---|---|---|---|
| 0=Déf II GK 25-87.5= Pool/Déf II 100= Pool GK | | | | | | |

### D) Sensibilité au taux de TAFI contenu dans l'échantillon de plasma

Des plasmas comprenant une concentration variable déterminée en TAFI ont été dosés selon la méthode décrite ci-dessus.

Il s'agit de :
- Plasma déficient en TAFI ;
- Plasmas d'un pool rendus plus ou moins déficients en TAFI ;
- Plasma d'un pool normal (pool GK) ;

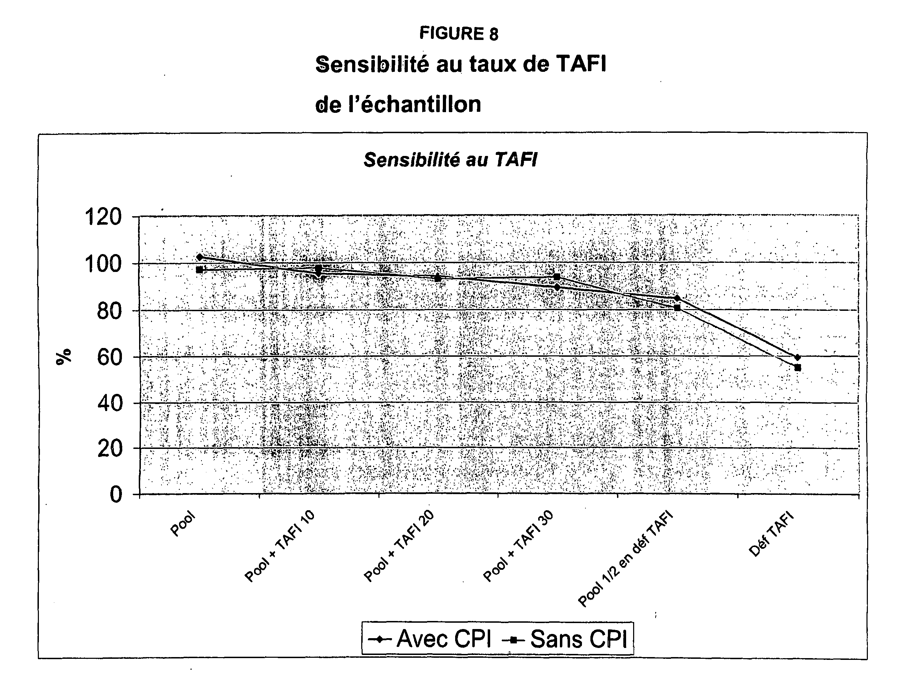

Les résultats (Tableau 9 et figure 8) montrent que le taux de TAFI de l'échantillon influence peu la valeur de l'activité fonctionnelle de la TM.

**Tableau 9**

| Sensibilité aux taux de TAFI de l'échantillon (1) | | | | | | | |
|---|---|---|---|---|---|---|---|
| Echantillons | | Pool+TAFI 10 | Pool + TAFI 20 | Pool + TAFI 30 | Pool | Pool ½ en def TAFI | Def TAFI |
| Avec CPI | DDO | 1.102 | 1.097 | 1.079 | 1.144 | 1.077 | 0.980 |
| | % | 95 | 94 | 89 | 103 | 85 | 59 |
| Sans CPI | DDO | 1.112 | 1.094 | 1.097 | 1.124 | 1.062 | 0.964 |
| | % | 98 | 93 | 94 | 97 | 81 | 55 |
| Δ | | -3 | 1 | -5 | 5 | 4 | 4 |

### E) Estimation de la stabilité

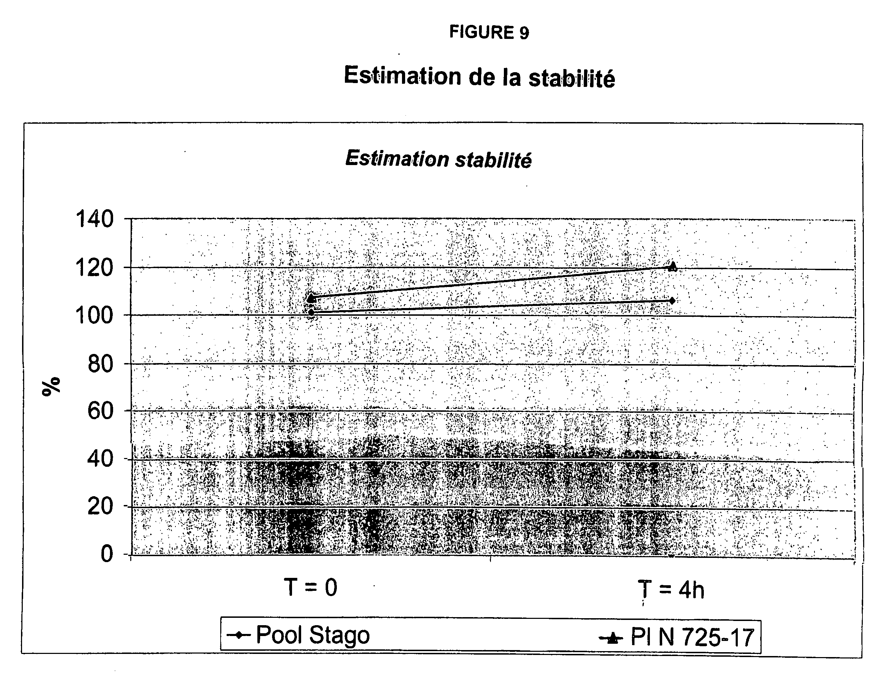

Le dosage de l'activité fonctionnelle de la TM a été reproduit dans le temps pour estimer la stabilité de la réaction. Le dosage réalisé à 4h d'intervalle sur un même échantillon a montré des valeurs correctement stables (Tableau 10 et figure 9).

**Tableau 10**

| | T=0 | | T=4h | | Ecart-relatif % |
|---|---|---|---|---|---|
| | DDO | % | DDO | % | |
| Pool Stago | 1.195 | 101 | 1.216 | 106 | 5 |
| PI N 725-17 | 1.220 | 107 | 1.274 | 121 | 13 |

PIN 725-17 est un plasma normal fourni par la société Manchester et correspondant au lot 725-17.

### F) Zones de lecture

La zone de lecture des densités optiques a été modifiée pour lire dans une fenêtre de 6-500 sec puis dans une fenêtre de 6-200 sec.

Les résultats obtenus sur différents plasmas étaient sensiblement équivalents (Tableau 11).

### IV Corrélation entre une augmentation de l'activité thrombomoduline et la perte précoce du foetus chez la femme enceinte.

La perte précoce du foetus est un problème clinique sérieux qui ne peut pas toujours expliqué adéquatement ou détecté. En dehors des causes anatomiques, chromosomiques ou des problèmes hormonaux, le syndrome anti-phospholipide (APS en anglais) est la seule cause commune qui peut être diagnostiquée jusqu'à présent. L'un des mécanismes proposés pour expliquer la fausse couche est l'hypoxie due à la thrombose utéro-placentaire. L'activité thrombomoduline peut être un marqueur important pour cette thrombose. Un test selon l'invention, de l'activité de la thrombomoduline, basé sur sa capacité à promouvoir l'activation de la protéine C par la thrombine, a été mis en oeuvre dans une étude de contrôle chez des patientes ayant subi une fausse couche précoce. Les résultats obtenus à partir d'échantillons de sang collectés durant le premier trimestre de grossesse chez les patientes (N=35), n'ayant pas d'historique de thrombose et ayant subi une fausse couche précoce (non liée à l'APS) ont été comparés avec les résultats obtenus à partir d'échantillons obtenus durant le premier trimestre de grossesse chez des femmes d'un groupe d'âge similaire ayant une grossesse normale (N=37) et avec des patientes normales qui n'étaient pas enceintes (N=32). Les échantillons de sang ont été collectés chez le groupe des patientes immédiatement après la fausse couche. Tous les échantillons de sang (1:10 dans 109 MN de citrate) ont été agités deux fois et le plasma a été congelé à -80°C jusqu'au test pour le test aPTT et pour l'activité thrombomoduline (Diagnostica Stago, France). Aucune modification dans le temps aPTT n'a été observée. Les valeurs moyennes du test de l'activité thrombomoduline étaient 106 ± 12% pour le contrôle versus 120 ± 10% pour les grossesses normales et 158 ±14% pour les fausses couches (p inférieur à 0,001). Bien que des études supplémentaires soient requises pour déterminer la capacité et la spécificité de ce test, la détermination de ce paramètre peut être un outil utile pour aider la détection des femmes à risque de fausse couche. L'activité thrombomoduline apparaît comme un marqueur prédictif précoce intéressant de la complication hypertensive de la grossesse qui, chez une femme à risque, peut permettre l'intervention pharmaceutique destinée à prévenir les manifestations cliniques les plus sévères.

**Tableau 11**

| | Echantillon normal | Grossesse normale | Fausse couche |
|---|---|---|---|
| | n= 32 | n=37 | n=35 |
| TM (activité) | 106 | 120 | 158 |
| | 65 - 139 | 69 - 152 | 77 - 279 |
| aPTT | 34.7 | 33.9 | 32.7 |
| | 29.4 - 39.6 | 29.5 - 40.4 | 28.6 - 37.3 |

### V Corrélation entre l'activité thrombomoduline et les complications de la grossesses.

Les situations de complications de la grossesse impliquent l'activité des cellules N. (natural killer) et la thrombose utéro-placentaire.

Les phospholipides procoagulants (PPL) peuvent moduler le rôle de l'activité NK et la TMa augmente dans le cas de dommages vasculaires.

Les mesures de la concentration en PPLs en utilisant un test de coagulation basé sur le facteur Xa (XaCT-Xa Clotting time) et la mesure de l'activité TMa peuvent présenter un intérêt puisque ces tests sont des marqueurs prédictifs pour la détection des femmes susceptibles de connaître une complication de la grossesse.

Des échantillons de sang obtenus chez des femmes (35), sans historique de thrombose, ayant rencontré des complications lors de la grossesse (non liées au Syndrome antiphospholipide), chez des femmes (37) d'un même groupe d'âge avec une grossesse normale et chez des femmes (32) sans pathologie identifiée et n'étant pas enceintes ont été testés. Les échantillons ont été traités pour recueillir le plasma qui a été conservé à -80°C avant test.

Les PPLs ont été mesurés dans un test XaCT. Un temps de coagulation raccourci reflète une élévation de la concentration en PPL. L'activité TMa a été mesurée dans un test chromogénique basé sur la capacité de promouvoir l'activation de la protéine C (comme décrit plus haut).

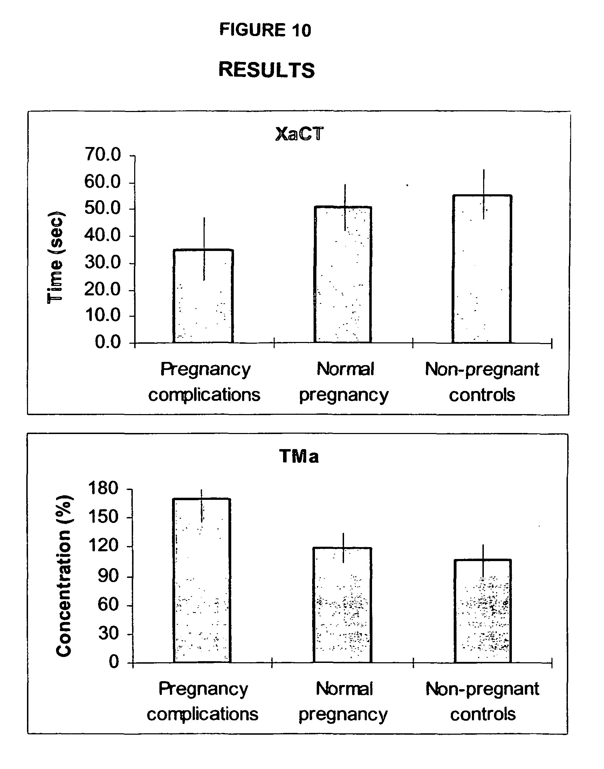

La comparaison des résultats obtenus (Figure 10) sur les 3 types d'échantillons montre : 1) pour les tests XaCT : des moyennes de 35,2 ± 11,8s ; 50,6 ± 8,6s et 55,5 ± 9,2s respectivement pour les patientes ayant une grossesse avec complications, une grossesse normale, et les contrôles (p< 0,001) ; 2) pour les tests d'activité de TMa : des moyennes de 169 % ± 24 , 119 % ± 16 et 106 % ± 16 respectivement pour les grossesses avec complications, les grossesses normales et les contrôles (p < 0,05).

Il apparaît donc qu'à la fois les tests XaCT et TMa ont un intérêt en tant que marqueurs prédictifs pour la détection des femmes ayant un risque de complication de leur grossesse.

### VI - Corrélation entre une augmentation de l'activité thrombomoduline et l'infarctus du myocarde

La mesure de l'activité thrombomoduline a été réalisée sur un pool de plasmas normaux et sur un échantillon de plasma prélevé chez un patient vivant souffrant d'infactus du myocarde (IDM) et sur un échantillon de plasma prélevé chez un patient décédé à la suite d'un IDM.

La mesure a été effectuée dans les conditions décrites au point I ci-dessus.

Les résultats montrent une augmentation sensible de l'activité de la thrombomoduline dans le cas d'IDM.

| | TM % |
|---|---|
| Normaux | 95 |
| Patient IDM vivant | 155 |
| Patient IDM décédé | 209 |

### VII - Corrélation entre l'activité thrombomoduline et l'allo-greffe de moëlle

L'activité thrombomoduline a été mesurée sur des échantillons de plasma prélevés chez des patients (enfants) ayant subi une allo-greffe de moëlle.

Le niveau d'activité TM après la greffe varie selon le stade du prélèvement d'échantillon après la greffe. On observe une différence dans l'évolution de l'activité de TM, selon que la greffe évolue avec des complications ou, au contraire, connaît une évolution favorable.

## Revendications

1. Méthode de mesure *in vitro* de l'activité fonctionnelle de la thrombomoduline, ladite méthode comprenant le dosage en milieu biologique, à partir d'un échantillon biologique, de l'activation de la protéine C en protéine C activée (PCa) par la thrombine en présence de son cofacteur ladite thrombomoduline, ladite méthode comprenant l'addition au plasma de l'échantillon, des agents nécessaires à l'activation du système de la protéine C, l'addition de protéine C purifiée et également l'addition d'un inhibiteur de la polymérisation de la fibrine.

2. Méthode de mesure *in vitro* de l'activité fonctionnelle de la thrombomoduline selon la revendication 1, **caractérisée en ce que** la thrombomoduline dosée est la thrombomoduline plasmatique, l'échantillon biologique étant un échantillon de plasma, le dosage étant réalisé en milieu plasmatique.

3. Méthode selon la revendication 1 ou 2, **caractérisée en ce que**, pour activer le système de la protéine C, on ajoute de la thrombine et des ions calcium.

4. Méthode selon l'une des revendications 1 à 3, **caractérisée en ce que** l'on détermine l'activité de la protéine C activée à l'aide d'un substrat enzymatique.

5. Méthode selon la revendication 4, **caractérisée en ce que** l'on dose
- l'activité amidolytique de la protéine C activée, au moyen d'un substrat chromogénique ou fluorométrique de la PCa, ou
- une protéine plasmatique de la coagulation dont l'inhibition dépend de la PCa, par exemple le facteur Va, le facteur VIIIa, ou
- le TAFIa ou un substrat du TAFIA.

6. Méthode selon la revendication 5, **caractérisée en ce que** l'on dose la libération de paranitroaniline par le substrat synthétique CBS 42.46, lorsque l'activité amidolytique de la protéine C activée est mesurée.

7. Méthode selon l'une quelconque des revendications 2 à 6, comprenant les étapes de :
a) mise en contact d'un échantillon de plasma avec un réactif 1 comprenant la thrombine, contenue dans un milieu permettant l'activation du système de la protéine C, en particulier comprenant des ions calcium, le réactif 1 comprenant en outre un inhibiteur de la polymérisation de la fibrine;
b) incubation du milieu constitué à l'étape a) avec un réactif 2 comprenant la protéine C purifiée, pendant un temps suffisant pour permettre son activation en PCa par la thrombine complexée à son cofacteur la thrombomoduline contenue dans l'échantillon de plasma ;
c) la mise en contact du milieu constitué à l'étape b) comprenant la protéine C activée, avec un substrat enzymatique de la PCa ;
d) la détection de la transformation du substrat de la PCa et,
e) optionnellement le dosage d'un contrôle interne.

8. Méthode selon l'une des revendications 2 à 7, **caractérisée en ce que** l'échantillon de plasma est dilué, par exemple dilué au 1/3.

9. Méthode selon l'une quelconque des revendications 1 à 8, **caractérisée en ce que** l'inhibiteur de la polymérisation de la fibrine est un inhibiteur polypeptidique, par exemple le H-GlyProArgPro-OH.AcOH (*GPRP.AcOH*) ou est un anticorps anti-fibrinogène.

10. Méthode selon l'une quelconque des revendications 7 à 9, **caractérisée en ce que** le tampon thrombine comprend essentiellement les composés suivants :
NaCl (0,15 mmol/l), Tris (20 mmol/l), CaCl2 (2,5 mmol/l), BSA (5mg/ml) et,
lorsque l'échantillon de plasma est caractéristique d'un patient traité par l'héparine, un inhibiteur de l'héparine, par exemple le polybrène (10g/l).

11. Méthode selon l'une quelconque des revendications 7 à 10, **caractérisée en ce que** le substrat enzymatique de la protéine C activée est l'oligopeptide THC-Pro-Arg-pNa.

12. Méthode selon l'une quelconque des revendications 7 à 11, **caractérisée en ce que** la réaction est effectuée dans les conditions suivantes :
- pour l'étape a), on met en contact 70 µl d'échantillon de plasma dilué au 1/3, avec 40 µl de thrombine 10 NIH diluée dans un tampon contenant NaCl (0,15 mmol/l), Tris (20 mmol/l), CaCl2 (2,5 mmol/l), BSA (5mg/ml) et en présence d'inhibiteur de la polymérisation de la fibrine, par exemple *GPRP.AcOH* (10g/l) et, lorsque l'échantillon de plasma est celui d'un patient traité par l'héparine, un inhibiteur de l'héparine, par exemple le polybrène (10g/l) ;
- pour l'étape b) 30 µl de protéine C incubée pendant 600s avec le milieu constitué à l'étape a) ;
- pour l'étape c) 110 µl de substrat enzymatique de la protéine C activée, la quantité de substrat enzymatique transformé étant déterminée par lecture optique dans une zone de 6 à 500 s, à une longueur d'onde de 405 nm.

13. Méthode selon l'une quelconque des revendications 1 à 12, **caractérisée en ce que** la mesure quantitative de la transformation du substrat enzymatique de la PCa se fait par lecture de densité optique et les valeurs de densité optique obtenues sont comparées à une courbe de calibration.

14. Méthode selon l'une quelconque des revendications 7 à 13, **caractérisée en ce que** le réactif 1 est la thrombine contenue dans un plasma déficient en thrombomoduline, auquel on a ajouté un inhibiteur de la polymérisation de la fibrine et, lorsque le plasma testé est caractéristique d'un patient traité par un anticoagulant, un inhibiteur dudit anticoagulant.

15. Kit pour la mesure fonctionnelle de l'activité de la thrombomoduline, en milieu biologique, notamment plasmatique, comprenant :
- un réactif 1 comprenant la thrombine diluée dans un tampon comprenant un inhibiteur de la polymérisation de la fibrine et comprenant du calcium ;
- un réactif 2 comprenant la protéine C purifiée ;
- et le cas échéant, un réactif 3 comprenant un substrat enzymatique de la protéine C activée et, optionnellement
- un contrôle interne et, optionnellement
- une courbe de calibration de l'activité fonctionnelle de la thrombomoduline.

16. Kit selon la revendication 15, **caractérisé en ce que** le réactif 1 comprend essentiellement les composés suivants : NaCl (0,15 mmol/l), Tris (20 mmol/l), CaCl2 (2,5 mmol/l), BSA (5mg/ml) et lorsque l'échantillon de plasma de patient traité par l'héparine, un inhibiteur de l'héparine, par exemple le polybrène (10g/l) et un inhibiteur de la polymérisation de la fibrine, par exemple le GPRP.AcOH utilisé à une concentration de 1 à 15 g/l.

17. Kit pour la mesure fonctionnelle de l'activité de la thrombomoduline, en milieu biologique, notamment plasmatique, comprenant :
- un réactif 1 comprenant la thrombine diluée dans un tampon comprenant un inhibiteur de la polymérisation de la fibrine et comprenant du calcium ;
- un réactif 2 comprenant le TAFI et, le cas échéant,
- un réactif 3 comprenant un substrat du TAFIa et, optionnellement
- un contrôle interne, et optionnellement
- une courbe de calibration de l'activité fonctionnelle de la thrombomoduline.

18. Méthode de détection *in vitro* d'une anomalie de la coagulation, comprenant la mesure de l'activité fonctionnelle de la thrombomoduline plasmatique selon l'une quelconque des revendications 2 à 14.

19. Méthode selon la revendication 18, comprenant la comparaison du niveau de l'activité fonctionnelle mesurée de la thrombomoduline, à une valeur normale de cette activité obtenue après dosage de l'activité fonctionnelle de la thrombomoduline sur un pool d'échantillons de plasma normaux, ou établie à partir de valeurs de l'activité fonctionnelle de la thrombomoduline mesurées sur des échantillons de plasma normaux dosés individuellement.

20. Méthode selon l'une quelconque des revendications 1 à 14 ou 18-19, ou kit selon l'une quelconque des revendications 15 à 17 pour la détection :
- d'une augmentation du taux de thrombomoduline associée à des symptômes de coagulation intravasculaires disséminées (CIVD), de diabète, de leucémie myéloïde chronique, d'insuffisance hépatique et rénale, de prééclampsie, de purpura thrombotique thrombocytopénique, de rickettsioses, de maladie de *Behçet,* d'homocystinurie, de fausse couche ou
- d'une diminution du taux de thrombomoduline associée à des symptômes d'hypertension artérielle pulmonaire ou à des mélanomes, ou
- d'une diminution du taux de thrombomoduline associée à une mutation du gène de la thrombomoduline.

## Claims

1. A method for *in vitro* measurement of the functional activity of thrombomodulin, said method comprising assaying, in a biological medium from a biological sample, activation of protein C to activated protein C (PCa) by thrombin in the presence of its cofactor, said thrombomodulin, said method comprising adding to the plasma sample the agents necessary for activation of the protein C system, adding purified protein C and also adding a fibrin polymerization inhibitor.

2. The *in vitro* thrombomodulin functional activity measurement method as claimed in claim 1, wherein the assayed thrombomodulin is plasmatic thrombomodulin, the biological sample being a plasma sample, the assay being carried out in a plasmatic medium.

3. The method as claimed in claim 1 or claim 2, wherein in order to activate the protein C system, thrombin and calcium ions are added.

4. The method as claimed in one of claims 1 to 3, wherein the activity of activated protein C is determined using an enzymatic substrate.

5. The method as claimed in claim 4, wherein is assayed:
- the amydolytic activity of the activated protein C, using a chromogenic or fluorometric substrate for PCa, or
- a plasmatic protein of coagulation, the inhibition of which depends on PCa, for example factor Va, factor VIIIa,
- TAFIa or a substrate for TAFIa.

6. The method as claimed in claim 5, wherein the liberation of para-nitroaniline by the synthetic substrate CBS 42.46 is assayed, when amydolytic activity of the activated protein C is measured.

7. The method as claimed in any one of claims 2 to 6, comprising the steps of:
a) bringing a plasma sample into contact with a reagent 1 comprising thrombin, contained in a medium which can activate the protein C system, in particular comprising calcium ions, the reagent 1 further comprising a fibrin polymerization inhibitor;
b) incubating the medium constituted in step a) with a reagent 2 comprising purified protein C, for a period which is sufficient to allow its activation to PCa by the thrombin complexed to its cofactor, namely the thrombomodulin contained in the plasma sample;
c) bringing the medium constituted in step b) comprising the activated protein C into contact with an enzymatic substrate for PCa;
d) detecting the transformation of the substrate for the PCa, and
e) optionally, further assaying an internal control.

8. The method as claimed in one of claims 2 to 7, wherein the plasma sample is diluted, for example diluted by 1/3.

9. The method as claimed in any one of claims 1 to 8, wherein the fibrin polymerization inhibitor is a polypeptide inhibitor, for example H-GlyProArgPro-OH.AcOH (GPRP.AcOH) or is an anti-fibrinogen antibody.

10. The method as claimed in any one of claims 7 to 9, wherein the thrombin buffer essentially comprises the following compounds:
NaCl (0.15 mmol/L), Tris (20 mmol/L), CaCl₂ (2.5 mmol/L), BSA (5 mg/ml); and
when the plasma sample is characteristic of a patient treated with heparin, a heparin inhibitor, for example polybrene (10 g/l).

11. The method as claimed in any one of claims 7 to 10, wherein the enzymatic substrate for the activated protein C is the oligopeptide THC-Pro-Arg-pNa.

12. The method as claimed in any one of claims 7 to 11, wherein the constituents employed in the reaction are used under the following conditions:
• for step a), 70 µl of plasma sample diluted to 1/3 is brought into contact with 40 µlof 10 NIH thrombin diluted in a buffer containing NaCl (0.15 mmol/L), Tris (20 mmol/L), CaCl₂ (2.5 mmol/L), BSA (5 mg/ml) and in the presence of a fibrin polymerization inhibitor, for example GPRP.AcOH (10 g/l) and, when the plasma sample derives from a patient treated with heparin, a heparin inhibitor, for example polybrene (10 g/l);
• for step b), 30 µl of purified protein C is incubated for 600 seconds with the medium constituted in step a);
• for step c), 110 µl of enzymatic substrate for the activated protein C, the quantity of transformed enzymatic substrate being determined by reading the optical density within a period of 6 to 500 s, at a wavelength of 405 nm.

13. The method as claimed in any one of claims 1 to 12, wherein the quantitative measurement for the transformation of the enzymatic substrate for the PCa is carried out by recording the optical density and the values obtained for the optical density are compared with a calibration curve.

14. The method as claimed in any one of claims 7 to 13, wherein the reagent 1 is thrombin contained in a thrombomodulin-deficient plasma, to which has been added a fibrin polymerization inhibitor and, when the test plasma is characteristic of a patient treated with an anticoagulant, an inhibitor of said anticoagulant.

15. A kit for functionally measuring thrombomodulin activity in a biological medium, especially a plasmatic medium, comprising:
• a reagent 1 comprising thrombin diluted in a buffer comprising a fibrin polymerization inhibitor and comprising calcium ions;
• a reagent 2 comprising purified protein C;
• if appropriate, a reagent 3 comprising an enzymatic substrate for activated protein C and, optionally,
• an internal control and, oprionnally,
• a calibration curve for the functional activity of thrombomodulin.

16. A kit as claimed in claim 15, wherein the reagent 1 essentially comprises the following compounds: NaCl (0.15 mmol/L), Tris (20 mmol/L), CaCl₂ (2.5 mmol/L), BSA (5 mg/ml) and when the plasma sample derives from a patient treated with heparin, a heparin inhibitor, for example polybrene (10 g/l), and a fibrin polymerization inhibitor, for example GPRP.AcOH used in a concentration of 1 to 15 g/l.

17. A kit for functionally measuring thrombomodulin activity in a biological medium, especially a plasmatic medium, comprising:
• a reagent 1 comprising thrombin diluted in a buffer comprising a fibrin polymerization inhibitor and comprising calcium;
• a reagent 2 comprising TAFI; and, if appropriate
• a reagent 3 comprising a substrate for TAFIa and, optionally,
• an internal control, and optionally
• a calibration curve for the functional activity of thrombomodulin.

18. A method for *in vitro* detection of a coagulation anomaly, comprising measuring the functional activity of plasmatic thrombomodulin as claimed in any one of claims 2 to 14.

19. The method as claimed in claim 18, comprising comparing the level of functional activity measured for thrombomodulin with a normal value for said activity obtained after assaying the functional activity of the thrombomodulin for a pool of normal plasma samples, or established from values for the functional activity of thrombomodulin measured on individually assayed normal plasma samples.

20. The method as claimed in any one of claims 1 to 14 or 18-19 or a kit as claimed in any one of claims 15 to 17, for the detection:
- of an increase in the level of thrombomodulin associated with symptoms of disseminated intravascular coagulation (DIVC), diabetes, chronic myeloid leukaemia, hepatic and renal insufficiency, preeclampsia, thrombotic thrombocytopenic purpura, rickettsioses, Behcet's disease, homocystinuria, and miscarriage, or
- of a reduction in the level of thrombomodulin associated with symptoms of pulmonary arterial hypertension or melanoma, or
- of a reduction in the level of thrombomodulin associated with a mutation in the gene for thrombomodulin.

## Patentansprüche

1. In-vitro Verfahren zur Bestimmung der funktionalen Aktivität von Thrombomodulin, welches umfasst : Dosieren in einem biologischen Milieu aus einer biologischen Probe der Aktivierung des Proteins C zum aktivierten Protein C (PCa) durch Thrombin in Gegenwart seines Co-Faktors Thrombomodulin, wobei das Verfahren umfasst: Hinzufügen von Substanzen zum Plasma der Probe, die für die Aktivierung des Systems des Proteins C notwendig sind, Hinzufügen von gereinigtem Protein C und auch Hinzufügen eines Inhibitors der Fibrinpolymerisierung.

2. In-vitro Verfahren zur Bestimmung der funktionalen Aktivität von Thrombomodulin nach Anspruch 1, **dadurch gekennzeichnet, dass** das dosierte Thrombomodulin plasmatisches Thrombomodulin ist, dass die biologische Probe eine Plasmaprobe ist und dass die Dosierung in plamatischem Medium durchgeführt wird.

3. Verfahren nach Anspruch 1 oder 2, **dadurch gekennzeichnet, dass** Thrombin und Calciumionen hinzufügt werden, um das System des Proteins C zu aktivieren.

4. Verfahren nach einem der Ansprüche 1 bis 3, **dadurch gekennzeichnet, dass** die Aktivität des aktivierten Proteins C mit einem enzymatischen Substrat bestimmt wird.

5. Verfahren nach Anspruch 4, **dadurch gekennzeichnet, dass**:
- die amidolytische Aktivität des aktivierten Proteins C mit einem chromogenen oder fluorometrischen Substrat des PCa dosiert wird; oder
- ein plasmatisches Koagulationsprotein dessen Inhibitierung von PCa anhängt, zum Beispiel Faktor Va, Faktor VIIIa dosiert wird; oder
- das TAFIa oder ein Substrat des TAFIas dosiert wird.

6. Verfahren nach Anspruch 5, **dadurch gekennzeichnet, dass** die Freisetzung von para-Nitroanilin aus dem synthetischen Substrat CBS 42.46 dosiert wird, wenn die amidolytische Aktivität des aktivierten Proteins C bestimmt wird.

7. Verfahren nach einem beliebigen der Ansprüche 2 bis 6, welches die folgenden Schritte umfasst:
a) Inkontaktbringen einer Plasmaprobe mit einem Reagenz 1 welches Thrombin enthält, in einem Medium, das die Aktivierung des Proteins C erlaubt, insbesondere Calcium-Ionen enthält, wobei das Reagenz 1 weiter einen Inhibitor der Fibrinpolymerisierung enthält;
b) Inkubieren des Mediums aus Schritt a) mit einem Reagenz 2, das gereinigtes Protein C enthält, über eine Zeitspanne, die ausreichend ist, dessen Aktivierung zu PCa mit Thrombin kombiniert mit dessen Co-Faktor Thrombomobulin in der Plasmaprobe zu ermöglichen;
c) Inkontaktbringen des Mediums der Stufe b) welches das aktivierte Protein C enthält mit einem enzymatischen Substrat des PCa;
d) Bestimmen der Umwandlung des PCa-Substrats; und
e) wahlweise Dosieren einer internen Kontrolle.

8. Verfahren nach einem der Ansprüche 2 bis 7, **dadurch gekennzeichnet, dass** die Plasmaprobe verdünnt wird, zum Beispiel verdünnt 1/3.

9. Verfahren nach einem der Ansprüche 1 bis 8, **dadurch gekennzeichnet, dass** der Inhibitor der Fibrinpolymerisierung ein Polypeptidinhibitor ist, zum Beispiel H-GlyProArgPro-OH.AcOH (GPRP.AcOH) oder ein anti-Fibrinogen Antikörper ist.

10. Verfahren nach einem der Ansprüche 7 bis 9, **dadurch gekennzeichnet, dass** der Thrombinpuffer die folgenden Komponenten enthält: NaCl (0.15 mmol/l), Tris (20 mmol/l), CaCl₂ (2,5 mmol/l), BSA (5 mg/ml) und, wenn die Plasmaprobe aus einem mit Heparin behandelten Patienten charakteristisch ist, einen Heparin-Inhibitor, zum Beispiel Polybrene (10g)1).

11. Verfahren nach einem der Ansprüche 7 bis 10, **dadurch gekennzeichnet, dass** das enzymatische Substrat des aktivierten Proteins C das Oligopeptid THC-Pro-Arg-pNa ist.

12. Verfahren nach einem der Ansprüche 7 bis 11, **dadurch gekennzeichnet, dass** die Reaktion unter den folgenden Bedingungen durchgeführt wird:
- für Schritt a) Inkontaktbringen von 70 µl einer 1/3 verdünnten Plasmaprobe mit 40 µl Thrombin 10 NIH, verdünnt in einem Puffer, der NaCl (0.15 mmo/l), Tris (20 mmol/l), CaCl₂ (2,5 mmol/l), BSA (5 mg/ml) enthält und, wenn die Plasmaprobe von einem mit Heparin behandelten Patient stammt, einen Heparin-Inhibitor, zum Beispiel das Polybrene (10g/l);
- für Schritt b) 30 µl Protein C welches während 600 s mit dem Medium des Schritt a) inkubiert wurde;
- für Schritt c) 110 µl des enzymatischen Substrats des aktivierten Proteins C, wobei die Menge des umgewandelten enzymatischen Substrats bestimmt wird durch optisches Auslesen in einem Bereich von 6 bis 500 s bei einer Wellenlänge von 405 nm.

13. Verfahren nach einem der Ansprüche 1 bis 12, **dadurch gekennzeichnet, dass** die quantitative Bestimmung der Umwandlung des enzymatischen PCa-Substrats durch Auslesen der optischen Dichte durchgeführt wird und dass, die erhaltenen Werte der optischen Dichte mit einer Kalibratierungskurve verglichen werden.

14. Verfahren nach einem der Ansprüche 7 bis 13, **dadurch gekennzeichnet, dass** das Reagenz 1 Thrombin in einem Thrombomodulin-armen Plasma ist, dem ein Inhibitor der Fibrinpolymerisierung hinzugefügt wurde und, wenn das getestete Plasma charakteristisch ist für einen mit einem Antikoagulans behandelten Patienten, einen Inhibitor des Antikoagulans.

15. Kit für die Bestimmung der funktionalen Aktivität von Thrombomodulin in einem biologischen, insbesondere plasmatischen Medium, welcher umfasst:
- ein Reagenz 1, enthaltend Thrombin, das in einem Puffer verdünnt ist, der einen Inhibitor der Fibrinpolymerisierung und Calcium enthält;
- ein Reagenz 2, welches gereinigtes Protein C enhtält;
- und, gegebenenfalls, ein Reagenz 3, welches ein enzymatisches Substrat des aktivierten Protein C enthält; und wahlweise
- eine interne Kontrolle und, wahlerweise,
- eine Kalibratierungskurve der funktionalen Aktivität von Thrombomodulin.

16. Kit nach Anspruch 15, **dadurch gekennzeichnet, dass** das Reagenz 1 im Wesentlichen die folgenden Komponenten enthält: NaCl (0.15 mmo/l), Tris (20 mmol/l), CaCl₂ (2,5 mmol/l), BSA (5 mg/ml) und, wenn die Plasmaprobe für einen mit Heparin behandelten Patient charakteristisch ist, einen Heparin-Inhibitor, zum Beispiel Polybren (10g/l), und einen Fibrinpolymerisierunginhibitor, zum Beispiel GPRP.AcOH welcher in einer Konzentration von 1 bis 15 g/l eingesetzt wird.

17. Kit zur Bestimmung der funktionalen Aktivität von Thrombomodulin in einem biologischem, insbesondere plasmatischen Medium, welcher unfasst:
- ein Reagenz 1, enthaltend Thrombin, das in einem Puffer verdünnt ist, der einen Inhibitor der Fibrinpolymerisierung und Calcium enthält;
- ein Reagenz 2, welches TAFI enthält; und, gegebenenfalls,
- ein Reagenz 3, welches ein enzymatisches Substrat des TAFIa enthält; und wahlweise
- eine interne Kontrolle, und wahlweise,
- eine Kalibrierungskurve der funktionalen Aktivität von Thrombomodulin.

18. In-vitro Verfahren zum Nachweiss einer Anomalie der Gerinnung, wobei die funktionale Aktivität des plasmatischen Thrombomodulins nach einem der Ansprüche 2 bis 14 bestimmt wird.

19. Verfahren nach Anspruch 18, umfassend einen Vergleich des bestimmten Niveaus der funktionalen Aktivität von Thrombomodulin mit einem normalen Wert dieser Aktivität, erhalten durch Dosieren der funktionalen Aktivität von Thrombomodulin in einem Pools normaler Plasmaproben, oder bestimmt anhand von Werten der funktionalen Aktivität von Thrombomodulin, die aus einzeln dosierten, normalen Plasmaproben bestimmt wurden.

20. Verfahren nach einem der Ansprüche 1 bis 14 oder 18 und 19 oder Kit nach einem der Ansprüche 15 bis 17 zur Bestimmung einer:
- Erhöhung des Thrombomudulin-Gehalts assoziiert mit Symptomen einer intravasculären disseminierten Koagulation (CIVD), Diabetes, chronischer myeloider Leukämie, Leber- und Niereninsuffizienz, Preclampsie, Thrombozyten-Purpura, Thrombozytopenie, Rickettsiose, Beçhet-Krankheit, Homocystinurea, Fehlgeburt; oder
- Senkung des Thrombomodulin-Gehalts, assoziiert mit Symptomen des Bluthochdrucks der Lungenarterien oder Melanomen, oder
- Senkung des Thrombomodlin-Gehalts, assoziiert mit einer Mutation des Thrombomodulingenes.
